# EUROPÄISCHE PATENTSCHRIFT

(11) **EP 0 702 000 B1**
(45) Veröffentlichungstag und Bekanntmachung des Hinweises auf die Patenterteilung: **20.01.1999**
(21) Anmeldenummer: 95113969.0
(22) Anmeldetag: 06.09.1995
(51) Int. Cl.: C07C 263/14, C07C 263/20, C07C 263/18, C07C 265/14

(54) **Verfahren zur Einstellung eines bestimmten Gehaltes an Eisen in Diphenylmethandiisocyanat oder in Polyisocyanat-Gemischen der Diphenylmethandiisocyanat-Reihe**
Process for adjusting a certain amount of iron in diphenylmethane diisocyanate or in polyisocyanate mixtures of the diphenylmethane diisocyanate series
Procédé pour le réglage d'un certain contenu de fer en diphénylméthane diisocyanate ou dans des mélanges de polyisocyanate de la série diphénylméthane diisocyanate

(30) Priorität: 19.09.1994 DE 4433262
(43) Veröffentlichungstag der Anmeldung: 20.03.1996
(73) Patentinhaber: BAYER AG, 51368 Leverkusen (DE)
(72) Erfinder: Müller, Heinz-Herbert, Dr., D-47809 Krefeld (DE); Puppe, Lothar, Dr., D-51399 Burscheid (DE); Waldau, Eckart, Dr., D-40597 Düsseldorf (DE)

(56) Entgegenhaltungen:
- GB-A- 2 207 671

## Beschreibung

Die vorliegende Erfindung betrifft ein Verfahren zur Einstellung eines bestimmten Gehaltes an Eisen in Diphenylmethandiisocyanat oder in Polyisocyanat-Gemischen der Diphenylmethandiisocyanat-Reihe.

Technische Mischungen aus Diphenylmethandiisocyanaten und technische Polyisocyanat-Gemische der Diphenylmethandiisocyanat-Reihe können Spuren an Eisen oder anderen Metallen enthalten, die bei der Herstellung durch Korrosion und/oder Erosion der Anlagenwerkstoffe in die Isocyanate gelangen. Die Massenanteile der in den technischen Isocyanat-Gemischen enthaltenen Metall(Eisen)-Verbindungen liegen im allgemeinen in Größenordnungen < 10 ppm. Die Isocyanat-Gemische können jedoch, insbesondere wenn sie in der Anfahrphase der Produktion hergestellt werden, erheblich höhere Massenanteile an Eisenanteilen aufweisen, die in Abhängigkeit von den Produktionsbedingungen z.T. sogar Werte von 1 000 ppm und mehr erreichen.

Da die Isocyanat-Gemische technisch hauptsächlich als Ausgangsmaterialien für die Herstellung von Polyurethan-Polyadditionsprodukten Verwendung finden und die Aktivität von Isocyanaten bei der Reaktion mit Hydroxy-Verbindungen durch Spuren an Eisen beeinflußt wird, werden für die Isocyanat-Gemische konstante, möglichst niedrige Spurengehalte an Eisen als notwendige Voraussetzung für eine reproduzierbare Polyurethan-Qualität gefordert.

Der Erfindung liegt die Aufgabe zugrunde, die Gehalte an Metallen, insbesondere Eisen, bei Diphenylmethandiisocyanat bzw. Polyisocyanat-Gemischen der Diphenylmethandiisocyanat-Reihe, wenn diese erhöhte Gehalte aufweisen, auf eine tolerierbare Größenanordnung einzustellen.

Überraschenderweise wurde gefunden, daß die Einstellung eines bestimmten Eisen-Gehaltes bei Diphenylmethandiisocyanat bzw. bei Polyisocyanat-Gemischen der Diphenylmethandiisocyanat-Reihe mit mikroporösen Adsorbentien mit einer inneren Oberfläche von mindestens 100 m²/g hervorragend gelingt.

Gegenstand der vorliegenden Anmeldung ist daher ein Verfahren zur Einstellung eines Eisen-Gehaltes in Diphenylmethandiisocyanat oder in Polyisocyanat-Gemischen der Diphenylmethandiisocyanat-Reihe auf einen Massenanteil von 0,1 bis 100, bevorzugt 0,2 bis 70, ganz besonders bevorzugt 0,5 bis 20 mg/kg Diphenylmethandiisocyanat oder Polyisocyanat-Gemisch, das dadurch gekennzeichnet ist, daß man das Diphenylmethandiisocyanat oder die Polyisocyanat-Gemische in Gegenwart von mikroporösen Adsorbentien mit einer inneren Oberfläche von mindestens 100 m²/g, bevorzugt mindestens 400 m²/g, insbesondere mindestens 1 000 m²/g, bei Temperaturen von 20 bis 200°C, bevorzugt 20 bis 140°C, und bei Drücken von 0,5 bis 100 bar, bevorzugt 1 bar bis 10 bar, gegebenenfalls in Anwesenheit eines organischen Lösungsmittels behandelt.

Das erfindungsgemäße Verfahren kann sowohl in kontinuierlicher als auch in diskontinuierlicher Fahrweise durchgeführt werden.

Für das erfindungsgemäße Verfahren geeignete mikroporöse Adsorbentien können folgende Materialien umfassen:
Aktivkohlen, amorphe Fällungskieselsäuren, pyrogene Kieselsäuren, Aktivtonerden, Kieselgele, Bleicherden, Kaoline, Bentonite, Sepiolithe, Attagalgitte, natürliche und synthetische Zeolithe. Besonders geeignet sind Aktivkohlen sowie amorphe Kieselsäuren.

Die innere Oberfläche der Adsorbentien wurde nach der BET-Methode bestimmt.

Bei dem erfindungsgemäßen Verfahren müssen die Adsorbentien in getrocknetem Zustand eingesetzt werden, um Reaktionen des Wassers mit den Isocyanaten zu vermeiden. Das Adsorbens kann den zu behandelnden Isocyanaten in der diskontinuierlichen Fahrweise zugesetzt werden und das Gemisch, um einen guten Kontakt des Adsorbens zu gewährleisten, intensiv gerührt werden. Alternativ kann die Behandlung auch kontinuierlich erfolgen, in dem man die zu behandelnden Isocyanate durch ein Bett des Adsorbens mit solcher Geschwindigkeit leitet, daß eine für die Adsorption der Metalle ausreichende Kontaktzeit erzielt wird. Die Kontaktzeit beträgt bei kontinuierlicher Fahrweise üblicherweise 0,5 min bis 200 min, vorzugsweise 0,5 min bis 120 min.

Die Menge an eingesetzten Adsorbentien beträgt im allgemeinen 0,1 bis 200 g, bevorzugt 0,5 g bis 100 g, bezogen auf 1 kg eingesetztes Isocyanat. Die Adsorbentien können sowohl einzeln als auch im Gemisch untereinander eingesetzt werden.

Die eingesetzte Menge an Adsorbentien, die Behandlungszeit, der Druck und die Temperatur sind, wenn die Behandlung im diskontinuierlichen Verfahren durchgeführt wird, abhängig von der Art der zu behandelnden Isocyanate und dessen Eisen-Gehalte, vom Typ des ausgewählten Adsorbens und seiner Aktivität sowie von der Verfahrensvariante, ob Lösungen oder unverdünnte Isocyanate eingesetzt werden. Bei der kontinuierlichen Durchführung des Verfahrens hängen Verweilzeit, Temperatur und Druck in gleicher Weise von den zu behandelnden Isocyanaten und ihrem Einsatz in unverdünnter oder verdünnter Form sowie von dem eingesetzten Adsorbens ab. Das Optimum der erwähnten Parameter läßt sich durch entsprechende Vorversuche ermitteln.

Die Behandlung der Diphenylmethandiisocyanate in Lösung bietet sich immer dann an, wenn die Diphenylmethandiisocyanate eine hohe Viskosität aufweisen.

Als inerte, organische Lösungsmittel eignen sich insbesondere Aromaten, bevorzugt chlorierte Aromaten. Die Menge an eingesetzten Lösungsmitteln kann leicht durch Vorversuche ermittelt werden.

Das nach dem erfindungsgemäßen Verfahren behandelte Diphenylmethandiisocyanat oder dessen Gemische können direkt als Ausgangsmaterialien für beispielsweise Polyurethan-Polyadditionsprodukte verwendet werden. Durch die erfindungsgemäße Behandlung besitzen die Diphenylmethandiisocyanate eine reproduzierbare Reaktivität gegenüber H-aciden-Komponenten.

Es ist überraschend, daß durch die erwähnten mikroporösen Adsorbentien die Einstellung eines bestimmten Metallgehaltes bei Diphenylmethandiisocyanaten gelingt, da andere adsorptive Reinigungsverfahren zur Einstellung eines Spurengehaltes an Eisen, wie die Behandlung der Diphenylmethandiisocyanate mit beispielsweise den bekannten Ionenaustauschern, Zeolithe oder bestimmten Steinmehlen, nicht gelingt bzw. nur mäßige Wirkung zeigen.

### Beispiele

Die folgenden Beispiele verdeutlichen das erfindungsgemäße Verfahren, ohne es jedoch auf diese Beispiele einzugrenzen.

### Beispiel 1

95 Teile eines technischen Polyisocyanat-Gemisches auf Basis von Diphenylmethandiisocyanat mit einer Viskosität von 200 mPas und einem Isocyanatgruppen-Gehalt von 31,5 % NCO, das einen Gehalt von 170 mg Fe/kg aufwies, wurden mit 5 Teilen Aktivkohle (Typ C40-4, Fa. Carbo Tech; getrocknet im Wasserstrahlvakuum bei 120°C) 2 h bei Raumtemperatur gerührt. Anschließend wurde die Aktivkohle über Druckfiltration abfiltriert. Der Fe-Gehalt des gereinigten Isocyanats betrug 46 mg Fe/kg Isocyanat.

### Beispiel 2

30 Teile des in Beispiel 1 eingesetzten technischen Polyisocyanat-Gemisches wurden in 70 Teilen Chlorbenzol gelöst. 200 Teile dieser Chlorbenzol-Lösung wurden mit 13 Teilen Aktivkohle (Aktivkohle z. Analyse, Fa. Merck, Artikel-Nr. 2186, getrocknet bei 120°C im Wasserstrahlvakuum) versetzt und 2 h bei Raumtemperatur gerührt. Anschließend wurde die Aktivkohle über ein Faltenfilter abfiltriert. Es wurde eine Lösung mit einem Fe-Gehalt von 0,8 mg/kg erhalten, was einem Gehalt von 2,6 mg Fe/kg Isocyanat entspricht.

### Beispiel 3

Die in Beispiel 2 eingesetzte Isocyanat-Lösung in Chlorbenzol wurde durch einen Umpumpreaktor mit einem Volumen von 100 ml, der mit 45 g Aktivkohle (Formkohle NORIT ROW 0,8 Supra, Fa. Norit, getrocknet bei 120°C im Wasserstrahlvakuum) gefüllt war, mit einer Umpumpmenge von ca. 61 pro h bei Raumtemperatur im Kreislauf gepumpt. Nach 2 h betrug der Fe-Gehalt der Lösung 3,6 mg/kg bzw. 11 mg Fe/kg Isocyanat.

### Beispiel 4

Behandlung von 200 Teilen der in Beispiel 2 eingesetzten Isocyanat-Lösung mit 13 Teilen Ultrasil VN 3 (amorpher Kieselsäure), Fa. Degussa, getrocknet bei 120°C im Wasserstrahlvakuum, analog Beispiel 2. Es wurde eine Isocyanat-Lösung mit einem Fe-Gehalt von 0,3 mg/kg erhalten, was einem Gehalt von 0,9 mg Fe/kg Isocyanat entspricht.

### Beispiel 5

Behandlung von 200 Teilen der in Beispiel 2 eingesetzten Isocyanat-Lösung mit 8 Teilen Durosil (amorpher Kieselsäure), Fa. Degussa, getrocknet bei 120°C im Wasserstrahlvakuum, analog Beispiel 2 bei 132°C (1 h). Es wurde eine Isocyanat-Lösung mit einem Fe-Gehalt von 0,2 mg/kg erhalten, was einem Gehalt von 0,6 mg Fe/kg Isocyanat entspricht.

## Patentansprüche

1. Verfahren zur Einstellung eines Eisengehaltes in Diphenylmethandiisocyanat oder in Polyisocyanat-Gemischen der Diphenylmethandiisocyanat-Reihe auf einen Masssenanteil von 0,1 bis 100 mg/kg Diphenylmethandiisocyanat oder Polyisocyanat-Gemisch, dadurch gekennzeichnet, daß man das Diphenylmethandiisocyanat oder die Polyisocyanat-Gemische in Gegenwart von mikroporösen Adsorbentien mit einer inneren Oberfläche von mindestens 100 m²/g bei Temperaturen von 20 bis 200°C und Drücken von 0,5 bar bis 100 bar gegebenenfalls in Anwesenheit eines organischen Lösungsmittels behandelt.

2. Verfahren nach Anspruch 1, dadurch gekennzeichnet, daß man Adsorbentien in Mengen von 0,1 g bis 200 g, bezogen auf 1 kg Isocyanat, einsetzt.

3. Verfahren nach Ansprüchen 1 und 2, dadurch gekennzeichnet, daß man als Adsorbentien Aktivkohle und/oder amorphe Kieselsäuren einsetzt.

4. Verfahren nach Ansprüchen 1 bis 3, dadurch gekennzeichnet, daß man als inerte, organische Lösungsmittel Aromaten einsetzt.

## Claims

1. Process for establishing an iron content in diphenylmethane diisocyanate or in polyisocyanate mixtures of the diphenylmethane diisocyanate series to a proportion by weight of 0.1 to 100 mg/kg of diphenylmethane diisocyanate or polyisocyanate mixture, characterised in that diphenylmethane diisocyanate or the polyisocyanate mixtures are treated in the presence of microporous adsorbents having an internal surface area of at least 100 m²/g at temperatures of 20 to 200°C and pressures of 0.5 to 100 bar, optionally in the presence of an organic solvent.

2. Process according to claim 1, characterised in that the adsorbents are used in quantities of 0.1 to 200 g, relative to 1 kg of isocyanate.

3. Process according to claims 1 and 2, characterised in that adsorbents used are activated carbon and/or amorphous silicas.

4. Process according to claims 1 to 3, characterised in that the inert, organic solvents used are aromatic compounds.

## Revendications

1. Procédé pour régler un diphénylméthanediisocyanate ou un mélange de polyisocyanates de la série du diphénylméthanediisocyanate à une teneur en fer de 0,1 à 100 mg/kg du diphénylméthanediisocyanate ou du mélange de polyisocyanates, caractérisé en ce que l'on traite le diphénylméthanediisocyanate ou le mélange de polyisocyanates en présence d'adsorbants microporeux ayant une surface interne d'au moins 100 m²/g à des températures de 20 à 200°C et des pressions de 0,5 à 100 bar, éventuellement en présence d'un solvant organique.

2. Procédé selon la revendication 1, caractérisé en ce que les adsorbants sont mis en oeuvre en quantité de 0,1 g à 200 g pour 1 kg d'isocyanate.

3. Procédé selon les revendications 1 et 2, caractérisé en ce que les adsorbants utilisés consistent en charbon actif et/ou silices amorphes.

4. Procédé selon les revendications 1 à 3, caractérisé en ce que l'on utilise en tant que solvants organiques inertes des solvants aromatiques.
